# EUROPEAN PATENT SPECIFICATION

(11) **EP 3 389 612 B1**
(45) Date of publication and mention of the grant of the patent: **29.07.2020**
(21) Application number: 16810404.0
(22) Date of filing: 16.12.2016
(51) Int. Cl.: A61K 8/36, A61K 8/41, A61Q 9/02, A61K 8/19, A61K 8/29, A61K 8/02

(54) **COMPOSITION INTENDED ESPECIALLY FOR SHAVING THE SKIN**
ZUSAMMENSETZUNG, INSBESONDERE ZUR RASUR DER HAUT
COMPOSITION NOTAMMENT DESTINEE AU RASAGE DE LA PEAU

(30) Priority: 17.12.2015 FR 1562627
(43) Date of publication of application: 24.10.2018
(73) Proprietor: L'OREAL, 75008 Paris (FR)
(72) Inventor: BOULEMDARAT, Khaled, 94152 Chevilly La Rue (FR); MEDARD, Fatma, 93400 Saint-Ouen (FR)
(74) Representative: Nony
(86) International application number: PCT/EP2016/081371
(87) International publication number: WO 2017/103060

(56) References cited:
- DE-A1- 3 246 797
- US-A- 5 209 925
- US-A- 6 147 040
- US-B1- 6 362 145

## Description

The present invention relates to the field of shaving keratin fibers, especially skin hairs and more particularly facial hair.

Shaving using an electric razor or trimmers has met with ever-increasing success in recent years, at the expense of shaving using a foaming composition and a manual razor.

However, there are still consumers who favor and prefer this method of shaving. In addition, there are also situations in which the use of an electric razor is not possible.

It is in this context that the present invention has been made.

The present invention relates to a novel shaving composition, which can be formulated in stick form and which falls within the category of nomad products that can be used in uncomfortable situations, especially in places lacking electricity, or even water.

Shaving compositions in the form of a stick are known in the prior art, such as, for example, from US 5 209 925.

The problem posed, which is the origin of the present patent application, is that of providing a composition that has a particular consistency, i.e. which is hard enough to be able to be formulated in the form of a stick, but soft enough to give a sufficient deposit and thus to be able to be used as a shaving product.

The composition according to the invention satisfies these expectations.

Thus, the present text describes a composition, in particular a cosmetic composition, which is especially intended for shaving the skin, comprising:
from 15% to 25% by weight of at least one C₁₆-C₂₀ fatty acid relative to the total weight of the composition,
from 1.5% to 2.7% by weight of sodium hydroxide relative to the total weight of the composition,
from 1% to 8% by weight of triethanolamine relative to the total weight of the composition.

According to a first aspect, the present invention is directed to a composition, in particular cosmetic composition, intended especially for shaving the skin, preferably facial skin, characterized in that it comprises:
from 15% to 25% by weight of at least one C16-C20 fatty acid relative to the total weight of the composition,
from 1.5% to 2.7% by weight of sodium hydroxide relative to the total weight of the composition,
from 1% to 8% by weight of triethanolamine relative to the total weight of the composition,
said composition having a hardness, measured at 25°C, ranging from 260 g to 1200 g.

The composition according to the invention has good properties in terms of hardness, covering power and stability.

Thus, the composition according to the invention satisfies the criteria of a formulation in stick form, and may thus be advantageously marketed in a compact form, without using propellant gas. The composition according to the invention may consequently be marketed using inexpensive and sparingly polluting packaging.

The formulation in stick form also allows application without contact with the user's hands, so the user therefore does not need to rinse his hands after the application.

The composition according to the invention also satisfies the required demands in terms of stability and covering power. It has thus been shown that three passes to and fro of the stick over the zone to be shaved are sufficient to allow the deposition of a sufficient amount of composition, this amount being capable of leading, optionally after massaging this zone, to a creamy foaming composition that allows shaving to be performed.

In addition, it has been observed that the composition according to the invention does not melt and does not crystallize: no appearance of crystals is observed on storage for two months at 45°C.

As emerges from the examples given below, the inventors have found that the compositions according to the invention have satisfactory hardness, covering power and stability, in accordance with the required demands.

According to another of its aspects, a subject of the present invention is also a shaving process, comprising the application to the skin, especially facial skin, of at least one composition in accordance with the invention.

In addition, the present invention also relates to the use of a composition in accordance with the invention as a shaving and optionally aftershave composition.

### FATTY ACID

The composition according to the invention comprises from 15% to 25% and preferably from 18% to 22% by weight of at least one C₁₆-C₂₀ fatty acid relative to the total weight of the composition.

Preferably, the C₁₆-C₂₀ fatty acid is a C₁₆-C₁₈ acid.

The C₁₆-C₂₀ fatty acid is advantageously chosen from palmitic acid, stearic acid, oleic acid, linoleic acid, linolenic acid and arachidic acid, and mixtures thereof; the C₁₆-C₂₀ fatty acid is particularly stearic acid.

Advantageously, the fatty acid used is of plant origin, i.e. it is not a synthetic acid.

### Sodium hydroxide

The composition according to the invention comprises from 1.5% to 2.7%, preferably from 1.8% to 2.5% and preferably from 2.0% to 2.3% by weight of sodium hydroxide (NaOH) relative to the total weight of the composition.

### Triethanolamine

The composition according to the invention comprises from 1% to 8%, preferably from 3.0% to 6.5% and preferably from 3.5% to 6.0% by weight of triethanolamine, also known as TEA, relative to the total weight of the composition.

The composition according to the invention is an emulsion obtained from two soaps, one based on sodium, the other based on TEA, derived from the *in-situ* neutralization of C₁₆- C₂₀ fatty acid(s).

The amount of base used, comprising, or even constituted by sodium hydroxide and triethanolamine, depends on the amount of fatty acid(s) present in the composition. A sufficient amount of base must be used to dissolve the fatty acid(s) in the aqueous phase and to produce a pH ranging from 4 to 8 and preferably from 5 to 7.

As already mentioned, the choice of particular components in astutely selected ranges allows the production of the composition according to the invention, which has the advantage of having adequate hardness and of giving a homogeneous, creamy deposit, in sufficient amount for use in shaving.

### Hardness

The composition according to the invention has a hardness, measured at 25°C, ranging from 260 g to 1200 g.

The texture of the composition may advantageously be characterized by a hardness, when it is subjected to a penetration test with a "cheese wire".

The object of the penetrometry measurement on the composition in stick form consists in determining the resistance force which opposes said composition during its penetration under defined conditions.

For the purposes of the present invention, the term "hardness" means the maximum penetration force obtained during the operation described below and expressed in grams.

It is measured at 25°C using a texturometer with software, such as the machine sold under the name TAXT2i or TA XTPlus by the company Rheo, equipped with a "cheese wire", by measuring the change in force (compressive force or penetration force) (F) as a function of time.

A sample of the composition to be characterized is introduced into packaging of "stick" type of oval cross section, the largest dimension of which is 103 mm and the smallest dimension of which is 48 mm, and 4 cm tall.

The sample is thermostatically maintained at 25°C. Three measurements are taken for the same composition, either at different locations evenly distributed and spaced out over the sample, or on different samples for the same composition. The average of these measurements indicates the hardness of the composition with a 95% confidence interval.

Thus, advantageously, a composition according to the invention may have, at a temperature of 25°C when it is measured using the texturometer sold under the name TA XTPlus by the company Rheo, a hardness ranging from 260 g to 1200 g, when it is subjected to the penetration at a force of 200 g and then 500 g, to a depth of 40 mm, of a spindle having the following specificities: pre-speed 0.5 mm/s, speed 0.5 mm/s, trigger force 2.0 g.

### Covering power

As already mentioned, the composition according to the invention must be hard enough for the product to be able to be formulated and stored in stick form, but the texture of the composition must also be capable of leading to a deposit sufficient to easily and uniformly cover the zone to be shaved.

It has thus been determined that an adequate deposit of the composition according to the invention may be obtained after three to and fro applications of the stick over the zone to be treated.

Needless to say, even though three to and fro applications are generally required, in certain cases one or two to and fro passes may suffice.

In addition, the application pressure of the stick on the zone to be treated corresponds to a standard application of a stick on the skin, for example an application of deodorant.

A deposit will be considered as sufficient if it enables masking of the zone to be shaved. Observation of the covering power is above all visual, i.e. after application of the composition, the skin onto which the composition has been applied is no longer visible.

Advantageously, the composition comprises at least one pigment for determining with the naked eye if the deposit on the zone of skin under consideration is sufficient to cover it correctly.

The term *"pigments"* means white or colored, mineral or organic particles, which are insoluble in an aqueous medium, and which are intended to color and/or opacify the resulting composition and/or deposit.

Advantageously, this pigment is a white pigment such as TiO₂; more particularly TiO₂ in untreated anatase form will be used.

Thus, the composition according to the invention comprises at least one pigment, preferably a white pigment such as TiO_{2.}

Preferably, the composition comprises from 0.01% to 20% by weight, especially from 0.1% to 10% by weight and in particular from 0.5% to 5% by weight of pigments relative to the total weight of said composition.

### Physiologically acceptable medium

Besides the compounds indicated previously, a composition according to the invention comprises a physiologically acceptable medium.

The term "physiologically acceptable medium" is intended to denote a medium that is particularly suitable for applying a composition of the invention to the skin and/or the lips, for instance water, the oils or organic solvents commonly used in cosmetic compositions.

The physiologically acceptable medium (acceptable tolerance, toxicology and feel) is generally adapted to the nature of the support onto which the composition is to be applied, and also to the form in which the composition is to be conditioned.

The composition according to the invention is an oil-in-water emulsion.

### Surfactants

The composition according to the invention comprises at least one surfactant, preferably a non-silicone surfactant. Preferably, the composition is such that the surfactant is present in a content ranging from 0.1% to 20% by weight relative to the total weight of the composition.

The composition according to the invention thus comprises at least one emulsifying surfactant appropriately chosen for obtaining an oil-in-water emulsion.

In particular, use may be made of an emulsifying surfactant having at 25°C an HLB balance (hydrophilic-lipophilic balance) within the Griffin sense of greater than or equal to 8.

An emulsifying surfactant having at 25°C an HLB balance (hydrophilic-lipophilic balance) within the Griffin sense of less than 8 may also be used.

This HLB value according to Griffin is defined in J. Soc. Cosm. Chem. 1954 (volume 5), pages 249-256.

These surfactants may be chosen from nonionic, anionic, cationic and amphoteric surfactants, and mixtures thereof. Reference may be made to Kirk-Othmer's Encyclopedia of Chemical Technology, volume 22, pages 333-432, 3rd Edition, 1979, Wiley, for the definition of the emulsifying properties and functions of surfactants, in particular pages 347-377 of this reference, for the anionic, amphoteric and nonionic surfactants.

Advantageously, the composition according to the invention comprises at least one anionic surfactant.

### Anionic surfactants

The anionic surfactants may be chosen from alkyl ether sulfates, carboxylates, amino acid derivatives, sulfonates, isethionates, taurates, sulfosuccinates, alkylsulfoacetates, phosphates and alkyl phosphates, polypeptides, metal salts of C₁₀-C₃₀ and especially C₁₆-C₂₅ fatty acids, and mixtures thereof, in particular metal stearates and behenates, and mixtures thereof.

Preferably, the anionic surfactant used is a salt of a C₆-C₂₄ alkyl ether sulfate comprising from 1 to 30 ethylene oxide units, in particular the alkali metal or alkaline-earth metal, ammonium, amine or amino alcohol salts and more particularly the sodium salts and more preferentially oxyethylenated sodium (C₁₂-C₁₄)alkyl ether sulfates comprising a mean number of OE units ranging from 1 to 4 and most particularly sodium laureth sulfate (CTFA name), more particularly the sodium lauryl ether sulfate (70/30 C12-14) (2.2 OE) sold under the name Sipon AOS225 or Texapon N702 by the company Henkel.

Mention may also be made of acyl isethionates such as sodium cocoylisethionate, such as the product sold under the name Jordapon CI P® by the company Jordan.

Advantageously, the composition comprises from 0.1% to 20%, preferably from 1% to 15% and preferably from 3% to 10% by weight of at least one anionic surfactant relative to the total weight of the composition.

The compositions according to the invention may also comprise at least one nonionic surfactant.

The nonionic surfactants may be chosen especially from alkyl and polyalkyl esters of poly(ethylene oxide), oxyalkylenated alcohols, alkyl and polyalkyl ethers of poly(ethylene oxide), optionally polyoxyethylenated alkyl and polyalkyl esters of sorbitan, optionally polyoxyethylenated alkyl and polyalkyl ethers of sorbitan, alkyl and polyalkyl glycosides or polyglycosides, in particular alkyl and polyalkyl glucosides or polyglucosides, alkyl and polyalkyl esters of sucrose, optionally polyoxyethylenated alkyl and polyalkyl esters of glycerol, and optionally polyoxyethylenated alkyl and polyalkyl ethers of glycerol, gemini surfactants, cetyl alcohol and stearyl alcohol, and mixtures thereof.

The compositions according to the invention may also comprise at least one cationic surfactant.

The cationic surfactants may be chosen from:
- alkylimidazolidiniums such as isostearylethylimidonium ethosulfate,
- ammonium salts such as (C₁₂₋₃₀ alkyl)tri(C₁₋₄ alkyl)ammonium halides, for instance N,N,N-trimethyl-1-docosanaminium chloride (or behentrimonium chloride).

The compositions according to the invention may also comprise at least one conditioning cationic polymer to improve the lubricity and the feel of the skin after shaving.

The composition according to the invention may thus have the properties of a shaving and aftershave composition.

The composition according to the invention advantageously comprises at least one C₆-C₁₀ aromatic ether of a C₂-C₉ polyol.

Preferably, the C₆-C₁₀ aromatic ether of a C₂-C₉ polyol is phenoxyethanol.

Advantageously, the composition comprises from 0.30% to 1% and preferably from 0.4% to 0.8% by weight, relative to the total weight of the composition, of at least one C₆-C₁₀ aromatic ether of a C₂-C₉ polyol.

### Oily phase

For the purposes of the invention, an oily phase comprises at least one oil.

The term *"oil"* means any fatty substance that is in liquid form at room temperature and atmospheric pressure.

An oily phase that is suitable for preparing the cosmetic compositions according to the invention may comprise hydrocarbon-based oils, silicone oils, fluoro oils or non-fluoro oils, or mixtures thereof.

The oils may be volatile or non-volatile.

They may be of animal, plant, mineral or synthetic origin.

For the purposes of the present invention, the term "non-volatile oil" is intended to mean an oil with a vapor pressure of less than 0.13 Pa.

A composition according to the invention may comprise from 1% to 95% by weight, better still from 1.5% to 40% by weight and preferably from 2% to 35% by weight of oil(s) relative to the total weight of said composition.

Advantageously, the compositions according to the invention comprise less than 5% by weight and preferably less than 3% by weight, and preferably are free of waxes.

The absence of waxes(es) makes it possible to avoid the risks of fouling of the razor blades.

### Aqueous phase

The aqueous phase of the composition according to the invention comprises water and optionally a water-soluble solvent.

In the present invention, the term *"water-soluble solvent"* denotes a compound that is liquid at room temperature and water-miscible (miscibility with water of greater than 50% by weight at 25°C and atmospheric pressure).

The water-soluble solvents that may be used in the composition of the invention may also be volatile.

Among the water-soluble solvents that may be used in the composition in accordance with the invention, mention may be made especially of lower monoalcohols containing from 1 to 5 carbon atoms such as ethanol and isopropanol, glycols containing from 2 to 8 carbon atoms such as ethylene glycol, propylene glycol, 1,3-butylene glycol and dipropylene glycol, C₃ and C₄ ketones and C₂-C₄ aldehydes.

The aqueous phase (water and optionally the water-miscible solvent) may be present in the composition in a content ranging from 5% to 95%, better still from 30% to 80% by weight and preferably from 40% to 75% by weight relative to the total weight of said composition.

According to another embodiment variant, the aqueous phase of a composition according to the invention may comprise at least one C₂-C₃₂ polyol.

For the purposes of the present invention, the term *"polyol"* should be understood as meaning any organic molecule comprising at least two free hydroxyl groups.

Preferably, a polyol in accordance with the present invention is present in liquid form at room temperature.

A polyol that is suitable for use in the invention may be a compound of linear, branched or cyclic, saturated or unsaturated alkyl type, bearing on the alkyl chain at least two -OH functions, in particular at least three -OH functions and more particularly at least four -OH functions.

The polyols that are advantageously suitable for formulating a composition according to the present invention are those especially containing from 2 to 32 carbon atoms and preferably 3 to 16 carbon atoms.

Advantageously, the polyol may be chosen, for example, from ethylene glycol, pentaerythritol, trimethylolpropane, propylene glycol, 1,3-propanediol, butylene glycol, isoprene glycol, pentylene glycol, hexylene glycol, glycerol, polyglycerols such as glycerol oligomers, for instance diglycerol, and polyethylene glycols, and mixtures thereof.

According to a preferred embodiment of the invention, said polyol is chosen from ethylene glycol, pentaerythritol, trimethylolpropane, propylene glycol, glycerol, polyglycerols, polyethylene glycols and mixtures thereof.

According to a particular mode, the composition of the invention may comprise at least propylene glycol.

According to another particular mode, the composition of the invention may comprise at least glycerol.

Advantageously, the polyol(s) are present in the composition in a content ranging from 0.1% to 40% by weight, especially from 1% to 20% by weight or even from 5% to 15% by weight relative to the total weight of said composition.

### Additive

Other additives may be used in the compositions according to the invention, such as moisturizers, also known as humectants, emollients, lubricants, refreshing agents and calmatives, silicones, vitamins, colorants other than pigments, antioxidants, antibacterial agents, antifungal agents or preserving agents.

Preferably, a moisturizer such as glycerol will be used.

The additive(s) are present in the composition in a content ranging from 0.1% to 20% by weight, especially from 0.5% to 15% by weight or even from 1% to 10% by weight relative to the total weight of said composition.

It is a matter of routine operations for those skilled in the art to adjust the nature and the amount of the additives present in the compositions in accordance with the invention so that the cosmetic properties desired for the latter are not affected thereby.

As mentioned, the present invention is also directed toward a shaving process comprising a step of applying the composition according to the invention to the skin, especially facial skin.

This step is followed by a step of shaving, advantageously using a razor, of the keratin fibers, in particular of the hairs of the zone onto which the composition has been applied.

As already mentioned, the application of the composition is ensured by performing one or more applications, one or more to and fro passes of the stick over the skin which has optionally been moistened beforehand. On conclusion of this application, the skin is masked with the composition.

The deposit obtained is foaming and creamy and is thus suitable for performing a shaving step using a mechanical razor.

Throughout the description, including the claims, the term *"comprising a"* should be understood as being synonymous with *"comprising at least one",* unless otherwise specified.

The terms *"between... and..."* and *"ranging from... to..."* should be understood as being inclusive of the limits, unless otherwise specified.

The invention is illustrated in greater detail by the examples presented below. Unless otherwise indicated, the amounts shown are expressed as mass percentages of active material (weight % relative to the total weight of the composition).

### EXAMPLES

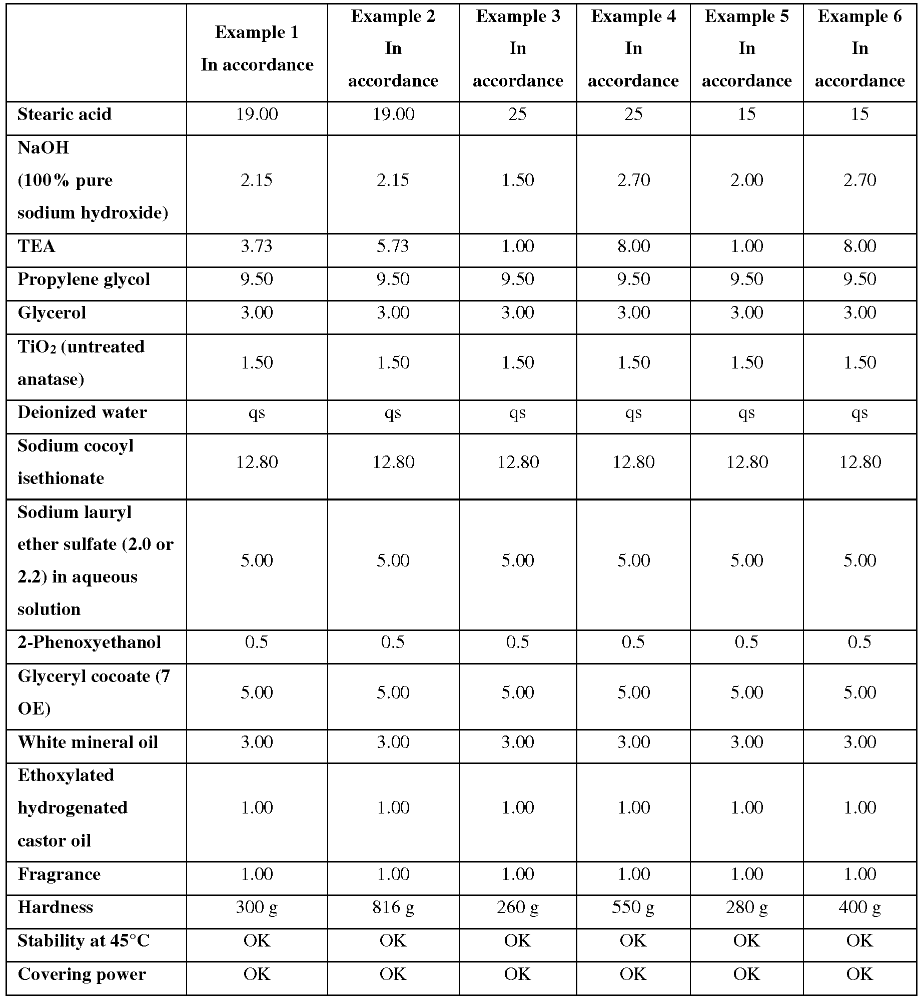

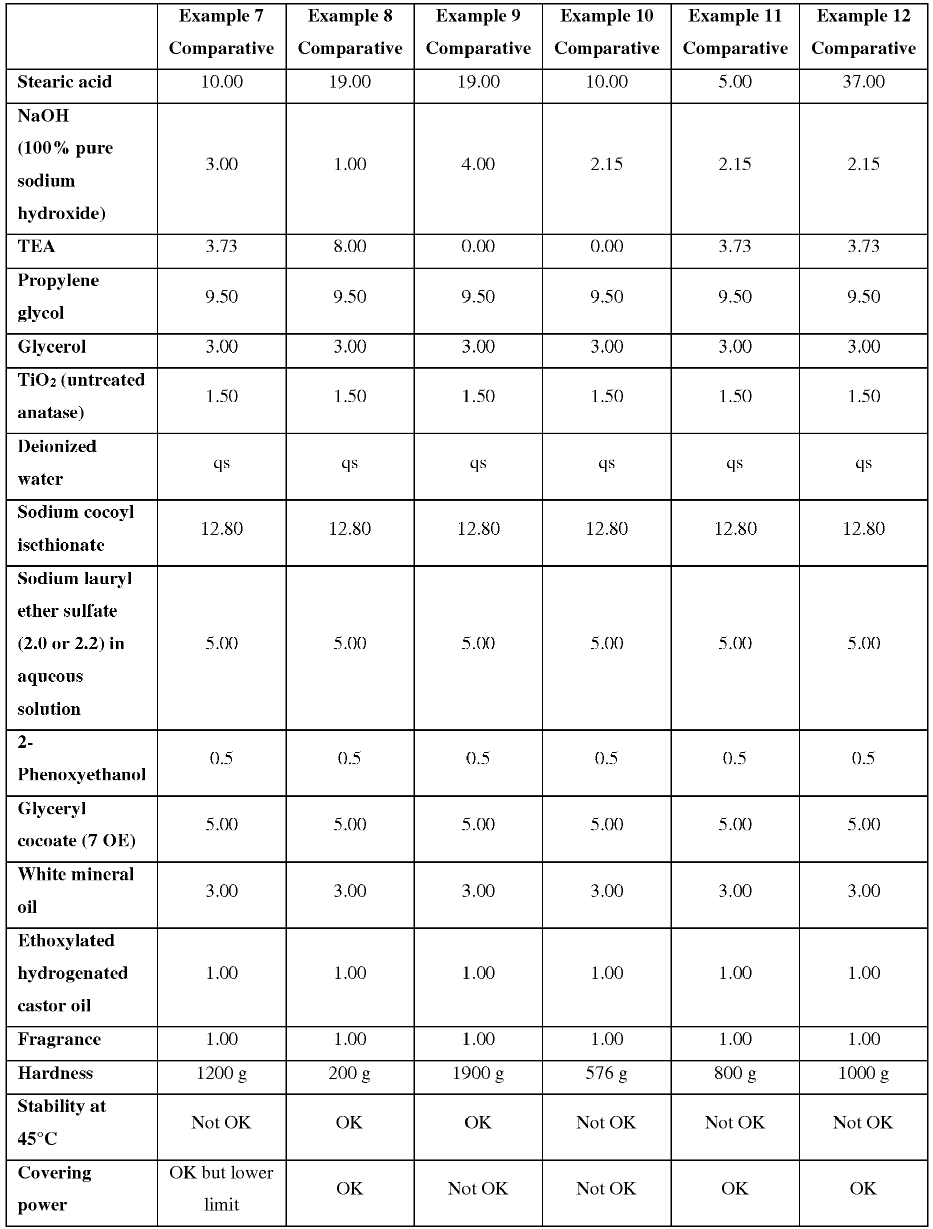

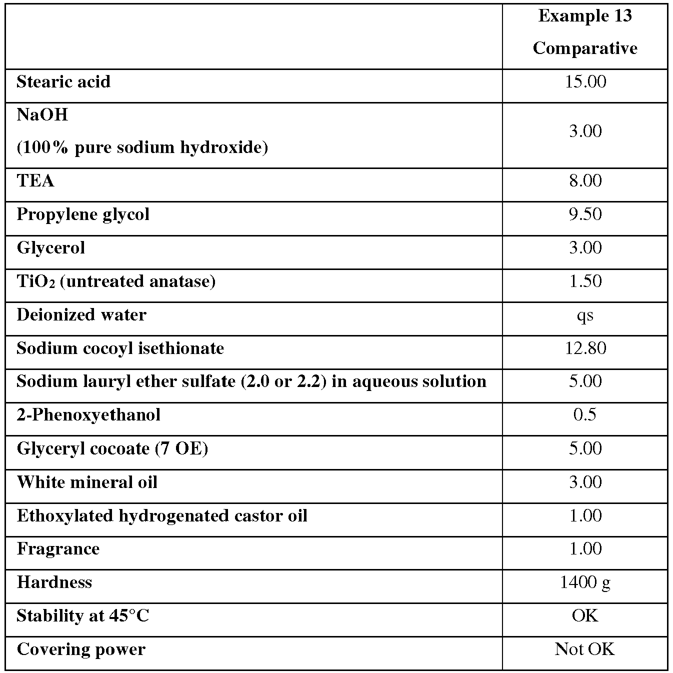

Only the compositions according to the invention lead to satisfactory results in terms of hardness, stability at 45°C and covering power.

## Claims

1. Composition, in particular cosmetic composition, intended especially for shaving the skin, preferably facial skin, **characterized in that** it comprises:
from 15% to 25% by weight of at least one C₁₆-C₂₀ fatty acid relative to the total weight of the composition,
from 1.5% to 2.7% by weight of sodium hydroxide relative to the total weight of the composition,
from 1% to 8% by weight of triethanolamine relative to the total weight of the composition,
said composition having a hardness, measured at 25°C, ranging from 260 g to 1200 g.

2. Composition according to the preceding claim, in which the C₁₆-C₂₀ fatty acid is chosen from palmitic acid, stearic acid, oleic acid, linoleic acid, linolenic acid and arachidic acid, and mixtures thereof, and is in particular stearic acid.

3. Composition according to one of the preceding claims, comprising at least one pigment, preferably a white pigment such as TiO_{2.}

4. Composition according to the preceding claim, in which comprising from 0.01% to 20% by weight, especially from 0.1% to 10% by weight and in particular from 0.5% to 5% by weight of pigments relative to the total weight of said composition.

5. Composition according to one of the preceding claims, comprising at least one anionic surfactant advantageously chosen from alkyl ether sulfates, carboxylates, amino acid derivatives, sulfonates, isethionates, taurates, sulfosuccinates, alkylsulfoacetates, phosphates and alkyl phosphates, polypeptides and metal salts of C₁₀-C₃₀ fatty acids, and mixtures thereof.

6. Composition according to the preceding claim, in which the anionic surfactant is a salt of a C₆-C₂₄ alkyl ether sulfate comprising from 1 to 30 ethylene oxide units, more preferentially oxyethylenated sodium (C₁₂-C₁₄)alkyl ether sulfates comprising a mean number of OE units ranging from 1 to 4, and most particularly sodium laureth sulfate.

7. Composition according to Claim 5 or 6, comprising from 0.1% to 20%, preferably from 1% to 15% and preferably from 3% to 10% by weight of at least one anionic surfactant relative to the total weight of the composition.

8. Composition according to one of the preceding claims, **characterized in that** it comprises less than 5% by weight of waxes, preferably less than 3% by weight of waxes, relative to the total weight of the composition, and preferably it is free of waxes.

9. Composition according to one of the preceding claims, comprising at least one polyol especially containing from 2 to 32 carbon atoms, in particular chosen from ethylene glycol, pentaerythritol, trimethylolpropane, propylene glycol, 1,3-propanediol, butylene glycol, isoprene glycol, pentylene glycol, hexylene glycol, glycerol, polyglycerols such as glycerol oligomers, for instance diglycerol, and polyethylene glycols, and mixtures thereof, and preferably the polyol is propylene glycol and/or glycerol.

10. Composition according to the preceding claim, in which the polyol(s) are present in the composition in a content ranging from 0.1% to 40% by weight, especially from 1% to 20% by weight or even from 5% to 15% by weight relative to the total weight of said composition.

11. Composition according to one of the preceding claims, also comprising at least one additive chosen from moisturizers, emollients, lubricants, refreshing agents and calmatives, silicones, vitamins, colorants other than pigments, antioxidants, antibacterial agents, antifungal agents and preserving agents.

12. Composition according to the preceding claim, in which the additive(s) are present in the composition in a content ranging from 0.1% to 20% by weight, especially from 0.5% to 15% by weight or even from 1% to 10% by weight relative to the total weight of said composition.

13. Shaving process, comprising the application to the skin, especially facial skin, of a composition according to one of the preceding claims.

14. Use of the composition according to any one of Claims 1 to 12, as a shaving and optionally aftershave composition.

## Patentansprüche

1. Zusammensetzung, insbesondere kosmetische Zusammensetzung, beabsichtigt insbesondere zur Rasur der Haut, bevorzugt Gesichtshaut, **dadurch gekennzeichnet, dass** sie umfasst:
15 Gew.-% bis 25 Gew.-% von mindestens einer C₁₆-C₂₀-Fettsäure, relativ zum Gesamtgewicht der Zusammensetzung,
1,5 Gew.-% bis 2,7 Gew.-% Natriumhydroxid, relativ zum Gesamtgewicht der Zusammensetzung,
1 Gew.-% bis 8 Gew.-% Triethanolamin, relativ zum Gesamtgewicht der Zusammensetzung,
wobei die Zusammensetzung eine Härte, gemessen bei 25°C, in einem Bereich von 260 g bis 1200 g aufweist.

2. Zusammensetzung gemäß dem vorhergehenden Anspruch, wobei die C₁₆-C₂₀-Fettsäure aus Palmitinsäure, Stearinsäure, Ölsäure, Linolsäure, Linolensäure und Arachinsäure und Gemischen davon ausgewählt ist, und insbesondere Stearinsäure ist.

3. Zusammensetzung gemäß einem der vorhergehenden Ansprüche, umfassend mindestens ein Pigment, bevorzugt ein Weißpigment wie TiO_{2.}

4. Zusammensetzung gemäß dem vorhergehenden Anspruch, welche 0,01 Gew.-% bis 20 Gew.-%, insbesondere 0,1 Gew.-% bis 10 Gew.-% und insbesondere 0,5 Gew.-% bis 5 Gew.-% Pigmente, relativ zum Gesamtgewicht der Zusammensetzung, umfasst.

5. Zusammensetzung gemäß einem der vorhergehenden Ansprüche, umfassend mindestens ein anionisches grenzflächenaktives Mittel, welches vorteilhafterweise aus Alkylethersulfaten, Carboxylaten, Aminosäurederivaten, Sulfonaten, Isethionaten, Tauraten, Sulfosuccinaten, Alkylsulfoacetaten, Phosphaten und Alkylphosphaten, Polypeptiden und Metallsalzen von C₁₀-C₃₀-Fettsäuren und Gemischen davon ausgewählt ist.

6. Zusammensetzung gemäß dem vorhergehenden Anspruch, wobei das anionische grenzflächenaktive Mittel ein Salz eines C6-C24-Alkylethersulfats, umfassend 1 bis 30 Ethylenoxideinheiten, stärker bevorzugt oxyethylenierte Natrium-(C₁₂-C₁₄)alkylethersulfate, umfassend eine mittlere Anzahl von OE-Einheiten in einem Bereich von 1 bis 4, und am stärksten besonders Natriumlaurethsulfat ist.

7. Zusammensetzung gemäß Anspruch 5 oder 6, umfassend 0,1 Gew.-% bis 20 Gew.-%, bevorzugt 1 Gew.-% bis 15 Gew.-% und bevorzugt 3 Gew.-% bis 10 Gew.-% von mindestens einem anionischen grenzflächenaktiven Mittel, relativ zum Gesamtgewicht der Zusammensetzung.

8. Zusammensetzung gemäß einem der vorhergehenden Ansprüche, **dadurch gekennzeichnet, dass** sie weniger als 5 Gew.-% Wachse, bevorzugt weniger als 3 Gew.-% Wachse, relativ zum Gesamtgewicht der Zusammensetzung, umfasst, und bevorzugt frei von Wachsen ist.

9. Zusammensetzung gemäß einem der vorhergehenden Ansprüche, umfassend mindestens ein Polyol, insbesondere enthaltend 2 bis 32 Kohlenstoffatome, insbesondere ausgewählt aus Ethylenglycol, Pentaerythritol, Trimethylolpropan, Propylenglycol, 1,3-Propandiol, Butylenglycol, Isoprenglycol, Pentylenglycol, Hexylenglycol, Glycerol, Polyglycerolen wie Glycerololigomeren, zum Beispiel Diglycerol, und Polyethylenglycolen, und Gemischen davon, und wobei bevorzugt das Polyol Propylenglycol und/oder Glycerol ist.

10. Zusammensetzung gemäß dem vorhergehenden Anspruch wobei die Polyol(e) in der Zusammensetzung in einem Gehalt in einem Bereich von 0,1 Gew.-% bis 40 Gew.-%, insbesondere 1 Gew.-% bis 20 Gew.-% oder sogar 5 Gew.-% bis 15 Gew.-%, relativ zum Gesamtgewicht der Zusammensetzung, vorhanden sind.

11. Zusammensetzung gemäß einem der vorhergehenden Ansprüche, auch umfassend mindestens ein Additiv, welches aus feuchtigkeitsspendenden Mitteln, Emollientien, Gleitmitteln, erfrischenden Mitteln und beruhigenden Mitteln, Siliconen, Vitaminen, farbgebenden Stoffen, welche verschieden von Pigmenten sind, Antioxidantien, antibakteriellen Mitteln, antimykotischen Mitteln und Konservierungsstoffen ausgewählt ist.

12. Zusammensetzung gemäß dem vorhergehenden Anspruch, wobei die Additiv(e) in der Zusammensetzung in einem Gehalt in einem Bereich von 0,1 Gew.-% bis 20 Gew.-%, insbesondere 0,5 Gew.-% bis 15 Gew.-% oder sogar 1 Gew.-% bis 10 Gew.-%, relativ zum Gesamtgewicht der Zusammensetzung, vorhanden sind.

13. Rasur, umfassend die Aufbringung einer Zusammensetzung gemäß einem der vorhergehenden Ansprüche auf die Haut, insbesondere Gesichtshaut.

14. Verwendung der Zusammensetzung gemäß einem der Ansprüche 1 bis 12 als eine Rasier- und gegebenenfalls Aftershave-Zusammensetzung.

## Revendications

1. Composition en particulier composition cosmétique, notamment destinée au rasage de la peau, de préférence la peau du visage, **caractérisée en ce qu'**elle comprend :
de 15 % à 25 % en poids d'au moins un acide gras en C₁₆-C₂₀ par rapport au poids total de la composition,
de 1,5 % à 2,7 % en poids d'hydroxyde de sodium par rapport au poids total de la composition,
de 1 % à 8 % en poids de triéthanolamine par rapport au poids total de la composition,
ladite composition ayant une dureté, mesurée à 25 °C, dans la plage de 260 g à 1200 g.

2. Composition selon la revendication précédente dans laquelle l'acide gras en C₁₆-C₂₀ est choisi parmi l'acide palmitique, l'acide stéarique, l'acide oléique, l'acide linoléique, l'acide linolénique et l'acide arachidique, et leurs mélanges, et est en particulier l'acide stéarique.

3. Composition selon l'une des revendications précédentes, comprenant au moins un pigment, de préférence un pigment blanc tel que TiO_{2.}

4. Composition selon la revendication précédente, comprenant de 0,01 % à 20 % en poids, notamment de 0,1 % à 10 % en poids, et en particulier de 0,5 % à 5 % en poids de pigments, par rapport au poids total de ladite composition.

5. Composition selon l'une des revendications précédentes, comprenant au moins un tensioactif anionique avantageusement choisi parmi des alkyléthersulfates, des carboxylates, des dérivés d'acide aminé, des sulfonates, des iséthionates, des taurates, des sulfosuccinates, des alkylsulfoacétates, des phosphates et alkylphosphates, des polypeptides et des sels métalliques d'acides gras en C₁₀-C₃₀, et leurs mélanges.

6. Composition selon la revendication précédente, dans laquelle le tensioactif anionique est un sel d'alkyléthersulfate en C₆-C₂₄ comprenant de 1 à 30 motifs oxyde d'éthylène, plus préférablement des (alkyle en C₁₂-C₁₄)éthersulfate de sodium oxyéthyléniques comprenant un nombre moyen de motifs OE dans la plage de 1 à 4, et tout particulièrement le laureth sulfate de sodium.

7. Composition selon la revendication 5 ou 6, comprenant de 0,1 % à 20 %, de préférence de 1 % à 15 % et de préférence de 3 % à 10 % en poids d'au moins un tensioactif anionique par rapport au poids total de la composition.

8. Composition selon l'une des revendications précédentes, **caractérisée en ce qu'**elle comprend moins de 5 % en poids de cires, de préférence moins de 3 % en poids de cires, par rapport au poids total de la composition et est de préférence exempte de cires.

9. Composition selon l'une des revendications précédentes, comprenant au moins un polyol contenant notamment de 2 à 32 atomes de carbone, en particulier choisi parmi l'éthylène glycol, le pentaérythritol, le triméthylolpropane, le propylène glycol, le 1,3-propanediol, le butylène glycol, l'isoprène glycol, le pentylène glycol, l'héxylène glycol, le glycérol, des polyglycérols tels que des oligomères de glycérol, par exemple le diglycérol, et des polyéthylène glycols, et leurs mélanges, et de préférence le polyol est le propylène glycol et/ou le glycérol.

10. Composition selon la revendication précédente, dans laquelle le(s) polyol(s) est/sont présent(s) dans la composition à une teneur dans la plage de 0,1 % à 40 % en poids, notamment de 1 % à 20 % en poids, ou même de 5 % à 15 % en poids, par rapport au poids total de ladite composition.

11. Composition selon l'une des revendications précédentes, comprenant en outre au moins un additif choisi parmi des agents hydratants, des émollients, des lubrifiants, des agents rafraîchissants et des agents apaisants, des silicones, des vitamines, des colorants autres que des pigments, des antioxydants, des agents antibactériens, des agents antifongiques et des agents conservateurs.

12. Composition selon la revendication précédente dans laquelle le ou les additif(s) sont présents dans la composition en une teneur allant de 0,1 % à 20 % en poids, notamment de 0,5 % à 15 % en poids, voire de 1 % à 10 % en poids, par rapport au poids total de ladite composition.

13. Procédé de rasage, comprenant l'application sur la peau, notamment la peau du visage, d'une composition selon l'une des revendications précédentes.

14. Utilisation de la composition selon l'une quelconque des revendications 1 à 12, en tant que composition de rasage et éventuellement d'après-rasage.
